# EUROPÄISCHE PATENTANMELDUNG

(11) **EP 2 725 350 A2**
(43) Veröffentlichungstag der Anmeldung: **30.04.2014**
(21) Anmeldenummer: 13189545.0
(22) Anmeldetag: 21.10.2013
(51) Int. Cl.: G01N 27/12, G01N 33/00

(54) **Verfahren zur Gasdetektion und entsprechende Gas-Sensorvorrichtung**

(30) Priorität: 23.10.2012 DE 102012110095
(71) Anmelder: Unitronic AG, 40472 Düsseldorf (DE)
(72) Erfinder: Schäfer, Eduard, 46519 Alpen (DE); Kundt, Stephan, 40547 Düsseldorf (DE); Haenel, Michael, 40476 Düsseldorf (DE)
(74) Vertreter: Michalski Hüttermann & Partner Patentanwälte

(57) **Zusammenfassung**

Verfahren zur Detektion mindestens eines oxidierbaren und/oder reduzierbaren Gases in einer eine Sensoroberfläche eines Halbleitergassensors (12), insbesondere eines Metalloxid-Halbleitergassensors, umgebenden Atmosphäre, die neben diesem mindestens einen zu detektierenden Gas auch ein weiteres Gas oder Gasgemisch als Hauptkomponente der Atmosphäre aufweist. Bei dem Verfahren sind folgende Schritte vorgesehen: (S1) Datenaufnahme einer Sensor-Messgröße des Halbleitergassensors (12) bei mehreren unterschiedlichen Sensortemperaturen in einem vorbestimmten Temperaturbereich, wobei der Halbleitergassensor (12) der Atmosphäre mit dem mindestens einen (zu detektierenden) oxidierbaren und/oder reduzierbaren Gas sowie dem die Hauptkomponente der Atmosphäre bildenden weiteren Gas oder Gasgemisch (zum Beispiel Luft) ausgesetzt ist. (S2) Normieren der aufgenommenen Daten mittels einer bereitgestellten temperaturabhängigen Normierungsgröße. (S3) Vergleich der so normierten Daten mit mindestens einem Datensatz normierter Daten der Sensor-Messgröße bei entsprechenden Sensortemperaturen für eine Atmosphäre mit mindestens einem oxidierbaren und/oder reduzierbaren Referenzgas bekannter Konzentration sowie dem die Hauptkomponente der Atmosphäre bildenden weiteren Gas oder Gasgemisch. (S4) Detektion des mindestens einen zu detektierenden Gases aufgrund der Vergleichsergebnisse als das Referenzgas oder zumindest eines der Referenzgase.

Weiterhin wird eine entsprechende Gas-Sensorvorrichtung (10) mit mindestens einem Halbleitergassensor (12) vorgestellt.

## Beschreibung

Die Erfindung geht aus von einem Verfahren zur Detektion mindestens eines oxidierbaren und/oder reduzierbaren Gases in einer eine Sensoroberfläche eines Halbleitergassensors, insbesondere eines Metalloxid-Halbleitergassensors, umgebenden Atmosphäre, die neben diesem mindestens einen zu detektierenden Gas auch ein weiteres Gas oder Gasgemisch als Hauptkomponente der Atmosphäre aufweist. Die Erfindung betrifft weiterhin eine entsprechende Gas-Sensorvorrichtung mit mindestens einem Halbleitergassensor, insbesondere Metalloxid-Halbleitergassensor, und mit einer Auswerteeinrichtung.

Seit 1962 sind Gassensoren (sogenannte TAGUCHI-Sensoren) bekannt, in welchen beheizte Schichten aus bestimmten Metalloxiden (z.B. Zinndioxid) der zu überwachenden Luft zum Zwecke der Detektion luftgetragener Gase oder Dämpfe ausgesetzt werden. Bei Begasung mit leichten oxidierbaren Gasen wie z.B. Wasserstoff dringt das Gas in die Metalloxidstruktur ein und reagiert mit dem Oxid, welches teilweise reduziert wird. Im Ergebnis vermindert sich der elektrische Widerstand des Metalloxids. Andere Gase oder Dämpfe adsorbieren zuerst an der Oberfläche der Wirkschicht und reagieren dann mit den Metalloxiden. Je nach Affinität der Gase oder Dämpfe zum als Wirkschicht eingesetzten Metalloxid und dessen Arbeitstemperatur und auch nach dessen Korngröße und Abstand der Kontaktelektroden zueinander, wird bei unterschiedlichen Gasen ein unterschiedliches Ansprechverhalten der Sensorwirkschicht auf das anwesende Gas festgestellt.

Eine Gas-Sensorvorrichtung mit einem Metalloxid-Halbleitergassensor der eingangs genannten Art ist zum Beispiel aus der WO 2006/089 789 A1 bekannt. Bei dieser Vorrichtung wird die sich im Sensor befindliche und sich gas- bzw. dampfabhängig ändernde elektrische Ladung als Maß für die Konzentration der in der umgebenden Atmosphäre des Sensors vorhandenen oxidierbaren oder reduzierbaren Gase oder Dämpfe genutzt und mittels einer Auswerteschaltung ausgewertet wird. Dabei ist die im Halbleitergassensor gespeicherte Ladung dadurch ermittelbar, indem der Sensor zuerst an einer Gleichspannungsquelle elektrisch geladen wird, wobei sich in der Wirkschicht des Sensors Ladungen aufbauen, und nach Beendigung des Ladungsvorgangs der Sensor stromlos geschaltet wird. Anschließend wird die Ladung über definierte Entladungsimpulse in einen Referenzkondensator entladen (Ladungsträger-Transfer-Verfahren), bevor innerhalb des Sensors eine Selbstentladung über die inneren ohmschen Anteile desselben stattfinden kann. Abhängig von der Größe des Referenzkondensators, dessen Lade-Spannung und eines gewählten Wertes einer Vergleichsspannung nach einer von der Ladung des Sensors abhängigen Zahl von Entladungsimpulsen wird die im Sensor gespeicherte elektrische Ladung bestimmt, wobei die Größe der elektrischen Ladung des Sensors (als Messgröße) davon abhängig ist, in welcher Atmosphäre der Sensor betrieben wird. Derartige Gas-Sensorvorrichtungen sind bisher vor Inbetriebnahme durch eine Kalibrierung ihrer jeweiligen Halbleitergassensoren mittels oxidierbarer/reduzierbarer Referenzgase (Kohlenmonoxid, Methan, etc.) individuell justiert worden.

Der Erfindung liegt die Aufgabe zugrunde, ein einfaches und effektives Verfahren zur Detektion mindestens eines oxidierbaren und/oder reduzierbaren Gases in einer Atmosphäre und eine entsprechende Gas-Sensorvorrichtung für diese Detektion bereitzustellen.

Die Lösung der Aufgabe erfolgt erfindungsgemäß durch die Merkmale der unabhängigen Ansprüche. Vorteilhafte Ausgestaltungen der Erfindung sind in den Unteransprüchen angegeben. Bei dem erfindungsgemäßen Verfahren sind folgende Schritte vorgesehen: (Schritt 1) Datenaufnahme einer Sensor-Messgröße des Halbleitergassensors bei mehreren unterschiedlichen Sensortemperaturen in einem vorbestimmten Temperaturbereich, wobei der Halbleitergassensor der Atmosphäre mit dem mindestens einen (zu detektierenden) oxidierbaren und/oder reduzierbaren Gas sowie dem die Hauptkomponente der Atmosphäre bildenden weiteren Gas oder Gasgemisch (zum Beispiel Luft) ausgesetzt ist. (Schritt 2) Normieren der aufgenommenen Daten mittels einer bereitgestellten temperaturabhängigen Normierungsgröße. (Schritt 3) Vergleich der so normierten Daten mit mindestens einem Datensatz normierter Daten der Sensor-Messgröße bei entsprechenden Sensortemperaturen für eine Atmosphäre mit mindestens einem oxidierbaren und/oder reduzierbaren Referenzgas bekannter Konzentration sowie dem die Hauptkomponente der Atmosphäre bildenden weiteren Gas oder Gasgemisch. (Schritt 4) Detektion des mindestens einen zu detektierenden Gases aufgrund der Vergleichsergebnisse des Vergleichs als das Referenzgas oder zumindest eines der Referenzgase.

Durch die Datenaufnahme der Messgröße des Halbleitergassensors bei mehreren unterschiedlichen Sensortemperaturen (Schritt 1) wird ein Sensor-Array nachgebildet. Der sich ergebende Datensatz der Daten zeigt ein charakteristisches "Muster" des oxidierbaren und/oder reduzierbaren Gases in der Atmosphäre, wobei das sich temperaturabhängig ergebende Muster aus diesem Datensatz weitgehend unabhängig von der Konzentration dieses Gases in der Atmosphäre ist. Einzig eine temperaturabhängige Sensitivität des Sensors geht bei diesem Muster noch mit ein. Die beim Normierungsschritt 2 bereitgestellte temperaturabhängige Normierungsgröße ist insbesondere eine Normierungsgröße, die eine Atmosphäre mit der reinen Hauptkomponente auf einen temperatur- und luftfeuchteunabhängigen konstanten Wert normiert.

Unter dem Begriff Gase können im Zusammenhang mit der Erfindung auch andere Fluide mit Gasanteil wie Dämpfe oder sogar Aerosole zu verstehen sein. Der Halbleitergassensor ist bevorzugt ein Metalloxid-Halbleitergassensor (MOX-Gassensor), wobei das Metalloxid beispielsweise Zinndioxid, Zinkoxid, Wolframoxid, Indiumoxid, Galliumoxid, etc. ist. Ein solcher Metalloxid-Halbleitergassensor, ist auch als TAGUCHI-Sensor bekannt. Zum Betrieb eines solchen Sensors ist dieser temperierbar, vorzugsweise elektrisch beheizbar. Das den Hauptanteil der Atmosphäre bildende Gas oder Gasgemisch ist dabei ein Trägergas oder Trägergasgemisch, das das mindestens eine oxidierbare und/oder reduzierbare Gas trägt. In der Regel ist Luft das den Hauptanteil der Atmosphäre bildende Gasgemisch. Dann ist das mindestens eine oxidierbare und/oder reduzierbare Gas ein luftgetragenes Gas.

Entscheidend ist bei dem vorliegenden Konzept, dass mit einem einzelnen Metalloxidsensor mehrere unterschiedliche Gase detektiert und erkannt werden können sofern deren charakteristische Muster bekannt sind. Gegenüber dem bekannten Detektionsverfahren mit einem Sensorarray, der eine Vielzahl von Metalloxidsensoren aufweist, hat das erfinderische Verfahren den Vorteil, dass Sensitivitätsunterschiede der verschiedenen Sensoren untereinander nicht ausgeglichen werden müssen.

Die Normierungsgröße lässt sich vorab durch eine Messung erstellen und die Daten und anschließend bereitstellen. Auch der mindestens eine Datensatz normierter Daten der Sensor-Messgröße bei entsprechenden Sensortemperaturen für eine Atmosphäre mit mindestens einen oxidierbaren und/oder reduzierbaren Referenzgas bekannter Konzentration sowie dem die Hauptkomponente der Atmosphäre bildenden weiteren Gas oder Gasgemisch lässt sich zuvor erstellen und für den Vergleich bei diesem Detektionsverfahren bereitstellen. Das mindestens eine Referenzgas gibt vor, auf welches Gas bzw. auf welche Gase man mit diesem Verfahren sensitiv ist. Die temperaturabhängige Normierungsgröße liegt beispielsweise in Form eines Datensatzes oder in Form einer mathematischen Funktion vor.

Durch das Verfahren mit diesen Verfahrensschritten wird der Aufwand bei der Kalibrierung des Sensors relativ gering gehalten. Ein Kalibrieren durch Messung mit dem zu detektierenden mindestens einen oxidierbaren und/oder reduzierbaren Gas ist nicht mehr unbedingt nötig, kann aber zur Verifikation selbstverständlich durchgeführt werden.

Gemäß einer bevorzugten Ausgestaltung des Verfahrens ist vorgesehen, dass die bereitgestellte temperaturabhängige Normierungsgröße zuvor durch die folgenden Schritte ermittelt wird:
(i) Datenaufnahme einer Sensor-Messgröße bei unterschiedlichen Sensortemperaturen des Halbleitergassensors oder eines baugleichen Halbleitergassensors in dem vorbestimmten Temperaturbereich, wobei der Halbleitergassensor der Atmosphäre mit dem reinen weiteren Gas oder Gasgemisch ausgesetzt ist, das die Hauptkomponente der Atmosphäre bildet, und
(ii) Normieren der aufgenommenen Daten auf einen temperatur- und luftfeuchteunabhängigen konstanten Wert unter Ermittlung der dazu notwendigen temperaturabhängigen Normierungsgröße. Das charakteristische Muster der reinen Hauptkomponente ist also eine Konstante (oder in einer Polardarstellung ein Kreis).

In einer bevorzugten Ausgestaltung der Erfindung werden die unterschiedlichen Sensortemperaturen insbesondere durch ein- oder mehrmaliges Durchfahren mindestens einer Temperaturmodulationsschleife eingestellt. Bei dieser Temperaturmodulationsschleife wird die Temperatur beispielsweise sinusförmig zwischen einem Maximalwert und einem Minimalwert der Temperatur moduliert.

In einer weiteren erfindungsgemäßen Ausgestaltung des Verfahrens ist vorgesehen, dass bei der Detektion des mindestens einen zu detektierenden Gases aufgrund der Vergleichsergebnisse auch dessen Konzentration bestimmt oder zumindest abgeschätzt wird.

Insbesondere ist vorgesehen, dass die Messgröße ein elektrischer Widerstand R des Halbleitergassensors oder eine in dem Halbleitergassensor befindliche Ladungsmenge (Menge der elektrischen Ladung Q) ist. Grundsätzlich kann der sich mit der Adsorption von unterschiedlichen Gasen an der Oberfläche einer Wirkschicht des Sensors ändernde Widerstand des Halbleitergassensors als Messgröße genutzt werden. Alternativ wird die in der Wirkschicht des Sensors eingespeicherte elektrische Ladungsmenge (Ladung Q) gemessen. Dabei wird der Halbleitergassensor mittels einer Spannungsquelle auf deren Spannungsniveau aufgeladen, sodass sich in der Wirkschicht des Sensors Ladungen aufbauen, und nach Beendigung des Ladungsvorgangs wird der Sensor stromlos geschaltet, wonach die nunmehr im Sensor gespeicherte elektrische Ladung messtechnisch mittels einer Auswerteeinrichtung (einer Auswerteschaltung) bestimmt wird, bevor innerhalb des Sensors eine Selbstentladung über die inneren ohmschen Anteile desselben stattfinden kann. Vorzugsweise ist die Spannungsquelle eine Gleichspannungsquelle.

In einer weiteren erfindungsgemäßen Ausgestaltung des Verfahrens ist vorgesehen, dass die Messgröße eine mittels Ladungsträger-Transferverfahren ermittelte Messgröße ist. Dabei erfolgt die Bestimmung der nach der Aufladung durch die (Gleich-)spannungsquelle im Sensor gespeicherten elektrischen Ladung durch eine Entladung des Sensors mit Hilfe des Ladungsträger-Transfer-Verfahrens, indem die elektrische Ladung sehr schnell in kleinen Ladungsmengen in einen Referenzkondensator übertragen wird, bevor innerhalb des Sensors eine Selbstentladung über die inneren ohmschen Anteile desselben stattfinden kann. Das bedeutet, dass mittels der Spannungsquelle der Sensor auf eine bestimmte Ladung aufgeladen wird, welche von der Spannung der Spannungsquelle und dem schon vorhandenen Ladungszustand der Wirkschicht des Sensors abhängt, wobei dieser Ladungszustand des Sensors von dem in der Atmosphäre enthaltenen reduzierbaren oder oxidierbaren Gas- oder Dampfkonzentrationen abhängt. Anschließend wird mittels des Ladungsträger-Transfer-Verfahrens die zusätzliche, mittels der (Gleich-)Spannungsquelle dem Sensor aufgegebene Ladung wieder abgeführt und in den Referenzkondensator übertragen, wobei diese vom Sensor abgegebene Ladung gemessen werden kann.

Bevorzugt wird zur Ermittlung der nach der Aufladung durch die Gleichspannungsquelle im Halbleitergassensor gespeicherten Ladung der Halbleitergassensor über definierte Entladungsimpulse in den Referenzkondensator entladen, wobei abhängig von der Größe eines Referenzkondensators und eines gewählten Schwellwertes nach einer von der Ladung des Sensors abhängigen Anzahl von Entladungsimpulsen die im Sensor gespeicherte elektrische Ladung bestimmt wird. Die Größe der elektrischen Ladung des Sensors ist dabei davon abhängig, in welcher Atmosphäre der Sensor betrieben wird.

Gemäß einer bevorzugten Ausgestaltung der Erfindung wird eine Datenaufnahme bei Atmosphäre mit dem reinen weiteren Gas oder Gasgemisch an einem temperatur- und Luftfeuchteunabhängigen konstanten Wert der normierten Daten erkannt und der Halbleitergassensor durch Anpassung der Normierungsgröße mittels dieser Daten kalibriert. Neben der Detektion von oxidierbaren und/oder reduzierbaren Gasen kann mittels des Verfahrens natürlich auch detektiert werden, dass kein solches oxidierbares und/oder reduzierbares Gas neben der Hauptkomponente in der Atmosphäre vorhanden ist. Die Daten des normierten Datensatzes entsprechen dann wieder einem temperatur- und Luftfeuchteunabhängigen konstanten Wert. Die Abweichung dieses Werts vom ursprünglich ermittelten Wert (aus Schritt ii) kann zur Sensorkalibrierung genutzt werden. So kann zum Beispiel ein Driften des Sensorsignals automatisch erkannt und durch aufaddieren oder subtrahieren eines Offsets korrigiert werden.

Gemäß einer noch weiteren bevorzugten Ausgestaltung der Erfindung ist die Ermittlung der temperaturabhängigen Normierungsgröße eine Ermittlung vor Erstinbetriebnahme einer den Halbleitergassensor aufweisenden Gas-Sensorvorrichtung.

Die Erfindung betrifft weiterhin eine Gas-Sensorvorrichtung mit mindestens einem Halbleitergassensor zur Detektion mindestens eines oxidierbaren und/oder reduzierbaren Gases in einer die Sensoroberfläche des Halbleitergassensors umgebenden Atmosphäre, mit einer Temperiereinrichtung zum gezielten Einstellen einer Sensortemperatur des Halbleitergassensors innerhalb eines Temperaturbereichs und mit einer eine Auswerteschaltung, einen Prozessor und einen Speicher aufweisenden Auswerteeinrichtung zur Auswertung einer Messgröße des Halbleitergassensors, wobei die Gas-Sensorvorrichtung insbesondere zur Detektion gemäß eines vorstehend genannten Verfahrens eingerichtet ist. Der Halbleitergassensor ist insbesondere als Metalloxid-Halbleitergassensor ausgebildet. Die Temperiereinrichtung ist bevorzugt eine Heizung, insbesondere eine elektrisch betriebene Heizung.

Insbesondere ist vorgesehen, dass die Temperiereinrichtung dazu eingerichtet ist, die Sensortemperatur des Halbleitergassensors in einer Temperaturmodulationsschleife zu durchfahren.

Generell kann die Gas-Sensorvorrichtung so ausgebildet sein, dass ein elektrischer Widerstand des Halbleitergassensors als Messgröße aufnehmbar ist. Mit Vorteil ist jedoch vorgesehen, dass die im Sensor befindliche und sich gas- oder dampfabhängig verändernde elektrische Ladungsmenge als Maß für die Konzentration der in der umgebenden Atmosphäre des Sensors vorhandenen oxidierbaren oder reduzierbaren Gase oder Dämpfe genutzt und mittels der Auswerteeinrichtung auswertbar ist.

Gemäß einer bevorzugten Ausgestaltung der erfindungsgemäßen Vorrichtung ist schließlich vorgesehen, dass die Auswerteeinrichtung einen Mikrocontroller, insbesondere einen für die Durchführung des Ladungsträger-Transferverfahren ausgelegten Mikrokontroller mit einer Schnittstelle für den direkten Anschluss des mindestens einen Halbleitergassensors, aufweist, wobei dieser Mikrocontroller den Prozessor umfasst. Ein solcher für die Durchführung des Ladungsträger-Transferverfahren ausgelegter Mikrokontroller mit einer Schnittstelle für den direkten Anschluss des Halbleitergassensors ist beispielsweise der Atmel AVR Controller. Dieser weist eine direkte Schnittstelle für QTouch®-Sensoren auf. Obwohl es sich bei diesen Sensoren um eine ganz andere Art von Sensoren handelt, wird jedoch ebenfalls das Ladungsträger-Transfer-(Charge-Transfer)-Verfahren genutzt. Bei einer Gas-Sensorvorrichtung mit Sensor-Array sind dann bevorzugt alle Halbleitergassensoren direkt an den Mikrocontroller angeschlossen.

Nachfolgend wird die Erfindung unter Bezugnahme auf die anliegenden Zeichnungen anhand bevorzugter Ausführungsformen näher erläutert. Es zeigen:
Fig. 1 eine Ausführungsform einer Gas-Sensorvorrichtung mit einem Halbleitergassensor gemäß einer bevorzugten Ausführungsform,
Fig. 2 ein Diagramm, bei dem die temperaturabhängigen Daten einer Normierte Messgröße über einzelnen Temperaturkanälen und der Konzentration des zu detektierenden Gases aufgetragen ist, sowie eine entsprechende Polardarstellung dieser Daten
Fig. 3 ein Diagramm, bei dem die Sensormessgröße für verschiedene Gase über der Sensortemperatur aufgetragen ist,
Fig. 4A - 4D weitere Polardarstellungen von Daten unterschiedlicher Gase und
Fig. 5 ein Flussdiagramm eines Verfahrens zur Detektion mindestens eines Gases in einer eine Sensoroberfläche des Halbleitergassensors.

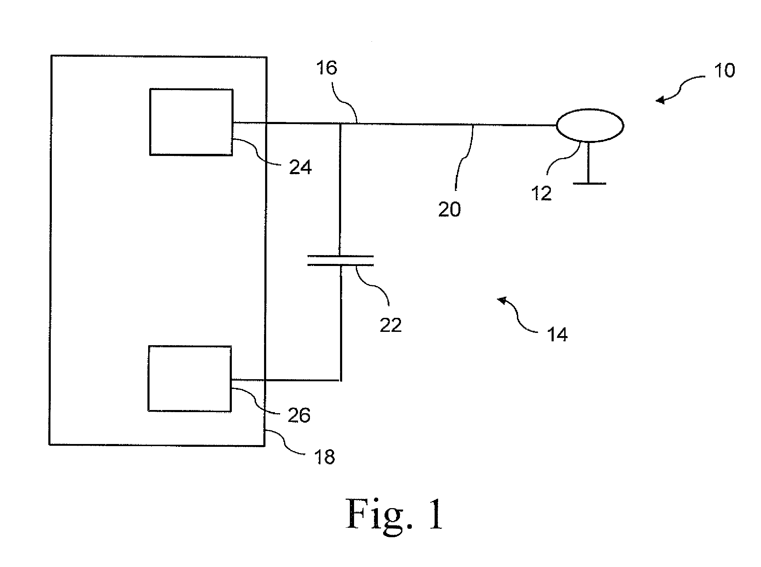

Eine bevorzugte Ausführungsform der Gas-Sensorvorrichtung 10 ist als schematischer Schaltplan in Fig. 1 gezeigt. Die Gas-Sensorvorrichtung 10 weist einen Halbleitergassensor 12 auf, der als Metalloxid-Halbleitergassensor zur Detektion eines oxidierbaren und/oder reduzierbaren Gases in einer die Sensoroberfläche dieses Halbleitergassensors 12 umgebenden Atmosphäre ausgebildet ist. Weiterhin weist die Gas-Sensorvorrichtung 10 eine Auswerteeinrichtung 14 auf. Diese umfasst eine Auswerteschaltung 16, einen Prozessor und einen Datenspeicher, wobei der Prozessor und der Speicher in einen Mikrocontroller 18 integriert sind. Die Auswerteeinrichtung 14 ist eine Auswerteeinrichtung zur Auswertung einer Messgröße des Halbleitergassensors 12, genauer gesagt einer in dem Halbleitergassensor 12 befindlichen Ladungsmenge (Menge der elektrischen Ladung Q). Alternativ könnte auch der sich mit der Adsorption der unterschiedlichen Gase an der Oberfläche einer Wirkschicht des Halbleitergassensors 12 ändernde Widerstand Rs des Halbleitergassensors 12 als Messgröße genutzt werden. Beide Messgrößen (Q und Rs) lassen sich jedoch ineinander überführen (umrechnen). Weiterhin weist die Sensorvorrichtung 10 eine Temperiereinrichtung, insbesondere eine Heizung auf (nicht gezeigt).

Bei der Aufnahme der elektrischen Ladungsmenge (Ladung Q) als Messgröße wird der Halbleitergassensor 12 mittels einer Spannungsquelle (nicht gezeigt) auf deren Spannungsniveau aufgeladen, sodass sich in der Wirkschicht des Sensors 12 Ladungen aufbauen. Nach Beendigung des Ladungsvorgangs wird der Sensor 12 stromlos geschaltet, wonach die nunmehr im Sensor 12 gespeicherte elektrische Ladung Q messtechnisch mittels der Auswerteschaltung 16 der Auswerteeinrichtung 14 bestimmt wird, bevor innerhalb des Sensors 12 eine Selbstentladung über die inneren ohmschen Anteile desselben stattfinden kann.

Im hier gezeigten Beispiel wird die Ladungsmenge Q mittels Ladungsträger-Transferverfahren ermittelt. Dabei erfolgt die Bestimmung der nach der Aufladung durch die (Gleich-)spannungsquelle im Sensor 12 gespeicherten elektrischen Ladung durch eine Entladung des Sensors 12 indem die elektrische Ladung über einen Strompfad 20 sehr schnell in kleinen Ladungsmengen in einen Referenzkondensator 22 übertragen wird, bevor innerhalb des Sensors 12 eine Selbstentladung über die inneren ohmschen Anteile desselben stattfinden kann. Das bedeutet, dass mittels der Spannungsquelle der Sensor 12 auf eine bestimmte Ladung Q aufgeladen wird, welche von der Spannung der Spannungsquelle und dem schon vorhandenen Ladungszustand der Wirkschicht des Sensors 12 abhängt, wobei dieser Ladungszustand des Sensors 12 von dem in der Atmosphäre enthaltenen reduzierbaren oder oxidierbaren Gas- oder Dampfkonzentrationen abhängt. Anschließend wird die zusätzliche, mittels der (Gleich-)Spannungsquelle dem Sensor aufgegebene Ladung Q wieder abgeführt und in den Referenzkondensator übertragen, wobei diese vom Sensor 12 abgegebene Ladung Q gemessen werden kann. Die Spannungsquelle wird im gezeigten Beispiel ebenfalls vom Mikrocontroller 18 realisiert.

Der Mikrocontroller 18 ist ein für die Durchführung des Ladungsträger-Transferverfahren ausgelegter Mikrokontroller mit einer Schnittstelle für den direkten Anschluss des mindestens einen Halbleitergassensors 12 über die Auswerteschaltung 16. Die Schnittstelle wird in der Fig. 1 durch die Ports 24, 26 repräsentiert. Ein solcher für die Durchführung des Ladungsträger-Transferverfahren ausgelegter Mikrokontroller 18 mit einer Schnittstelle für den direkten Anschluss des Halbleitergassensors ist beispielsweise der Atmel AVR Controller. Dieser weist eine direkte Schnittstelle für QTouch®-Sensoren auf. Obwohl es sich bei diesen Sensoren um eine ganz andere Art von Sensoren handelt, wird jedoch ebenfalls das Ladungsträger-Transfer-(Charge-Transfer)-Verfahren genutzt.

An dieser Stelle sei das Funktionsprinzip der Charge-Transfer-Messung noch einmal mit anderen Worten beschrieben:

Es gibt zwei als Kondensatoren wirkende Elemente 12, 22 die als Speicher für Ladungen Q dienen. Der Referenzkondensator 22 als Speicher und der Sensor 12 als Kondensator gegen Erde. Am Anfang werden beide entladen, um die Kondensatoren 12, 22 in einen definierten Zustand zu bringen. Das bedeutet, dass der Port 24 (PB1) und der Port 26 (PC1) als Ausgänge und auf LOW geschaltet werden. Nach kurzer Zeit ist die Entladung vollständig erfolgt. Danach werden in einer Schleife folgende Punkte abgearbeitet:
1. Der Sensor 12 wird über Port 24 aufgeladen (nur wenige Picofarad). Dabei wird der Port 26 als Eingang geschaltet damit in den Referenzkondensator 22 kein Strom fließen kann.
2. Port 24 wird als Eingang konfiguriert und Port 26 wird als Ausgang mit Potential 0V konfiguriert. Jetzt wandert die Ladung Q aus dem Sensor 12 in den Referenzkondensator 22 (welcher eine viel größere Kapazität aufweist als der Sensor 12 und sich daher nur geringfügig auflädt).
3. Wenn die Spannung am Referenzkondensator 22 ausreicht um vom Mikrocontroller 18 als eins ("High") gelesen zu werden, wird die Schleife beendet und durch geführte Schleifenanzahl ausgeben.

Der Zählerstand ist ein Maß für die benötigten Ladezyklen. Wenn nicht, beginnt die Schleife von vorne. Der Endstand des Zählers verändert sich, wenn sich in dem Sensor 12 die Werte durch Gasaufkommen verändern. Durch die kleinere Kapazität des Sensors 12 wird sozusagen schrittweise der große Referenzkondensator 22 aufgeladen. Die Anzahl der benötigten Ladezyklen hängt von der Kapazität des Sensors 12 ab. Und diese hängt wiederum von dem Messwert R_{S} des Sensors ab, der wiederum von der Gaskonzentration abhängig ist.

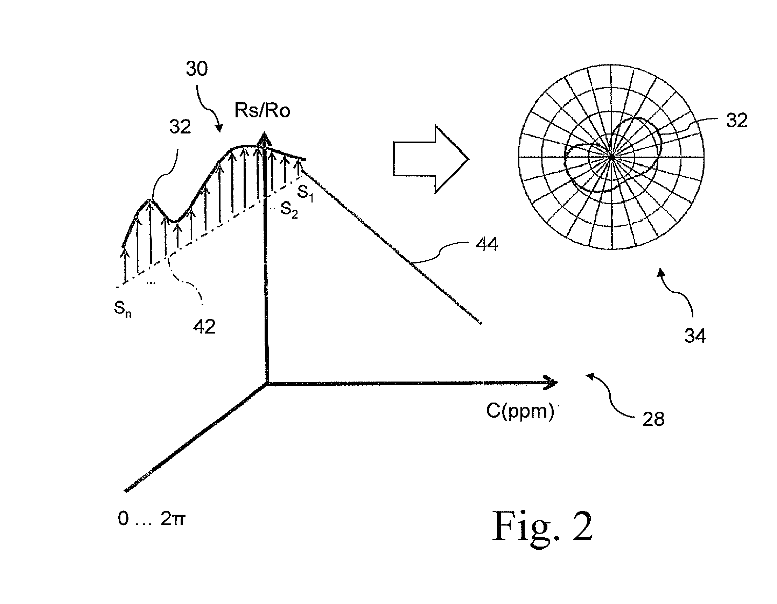

Die Fig. 2 zeigt links ein Diagramm 28, bei dem die Daten einer temperaturabhängig mittels des Halbleitergassensors 12 aufgenommenen und mittels einer temperaturabhängigen Normierungsgröße normierten Sensor-Messgröße (hier des Widerstandes R_{S}/R₀) dargestellt sind. Die unterschiedlichen Sensortemperaturen sind dabei durch ein- oder mehrmaliges Durchlaufen mindestens einer Temperaturmodulationsschleife 30 eingestellt worden. Jedem einzelnen normierten Datenpunkt ist dabei ein jeweiliger Kanal S₁, S₂, ..., Sₙ zugeordnet. Diese n Kanäle S₁, S₂, ..., Sₙ entsprechen beispielsweise einem sinusförmigen Durchlauf eines Temperaturintervalls in einem Bereich zwischen einem Maximalwert Tₘₐₓ und einem Minimalwert Tₘᵢₙ über die vollen 360° (bzw. 2π) der beispielsweise sinusförmigen Modulation. Das sich aus den Kanälen ergebende Muster 32 ist auf der rechten Seite der Fig. 2 in ein Polardiagramm 34 übertragen dargestellt. Der Temperaturbereich liegt typischerweise bei 100°C bis 500°C.

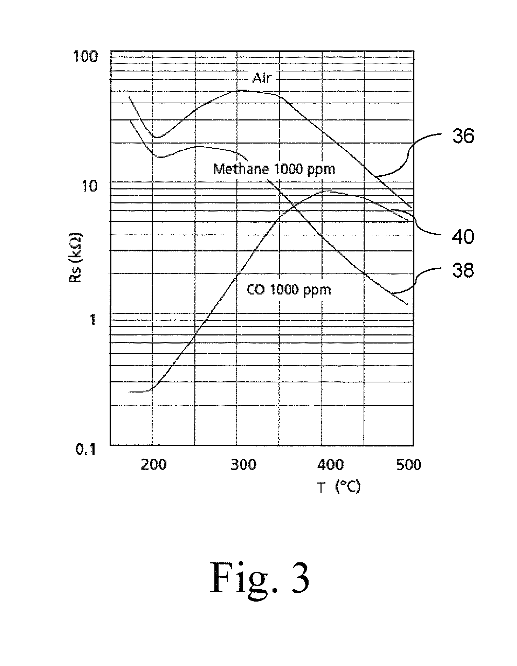

Zur Verdeutlichung der Ursache für die gasspezifische Charakteristik der Muster ist in Fig. 3 die Sensormessgröße Rs über der Sensortemperatur in einem Temperaturbereich von 175 °C bis knapp 500°C für unterschiedliche Gase gezeigt. Ein erster Funktionsgraph 36 zeigt die Abhängigkeit der Sensormessgröße, hier des elektrischen Widerstands Rs, von der Sensortemperatur im beschriebenen Temperaturbereich bei einer den Halbleitergassensor 12 umgebenden Atmosphäre reiner Luft. Ein zweiter Funktionsgraph 38 zeigt die Abhängigkeit der Sensormessgröße Rs von der Sensortemperatur bei einer den Halbleitergassensor 12 umgebenden Atmosphäre mit 1000 ppm Methan in der Luft. Ein dritter Funktionsgraph 40 zeigt schließlich die Abhängigkeit der Sensormessgröße Rs von der Sensortemperatur bei einer den Halbleitergassensor 12 umgebenden Atmosphäre mit 1000 ppm Kohlenmonoxid (CO) in der Luft. Der Kurvenverlauf jeder dieser drei Funktionsgraphen 36, 38, 40 ist trotz der relativ geringen Beimengung von zu detektierendem Gas (Methan beziehungsweise Kohlenmonoxid) zu dem die Hauptkomponente Luft bildenden Gasgemisch sehr charakteristisch und unterscheidet sich deutlich von den Kurvenverläufen der jeweils anderen Funktionsgraphen.

Die Normierungsfunktion wurde dazu zuvor mittels folgender Verfahrensschritte ermittelt:

Eine Datenaufnahme bei Durchlaufen einer Temperaturmodulationsschleife der Sensor-Messgröße eines baugleichen Halbleitergassensors in dem vorbestimmten Temperaturbereich, wobei der baugleiche Halbleitergassensor der Atmosphäre mit der reinen Hauptkomponente der Atmosphäre (zum Beispiel reiner Luft) ausgesetzt wurde. Weiterhin ein Normieren der aufgenommenen Daten auf einen temperatur- und luftfeuchteunabhängigen konstanten Wert für die Atmosphäre mit der reinen Hauptkomponente unter Ermittlung der dazu notwendigen temperaturabhängigen Normierungsgröße. Diese Normierungsgröße wird dann zum Normieren der Daten für die Atmosphäre mit zu detektierendem Gas genutzt.

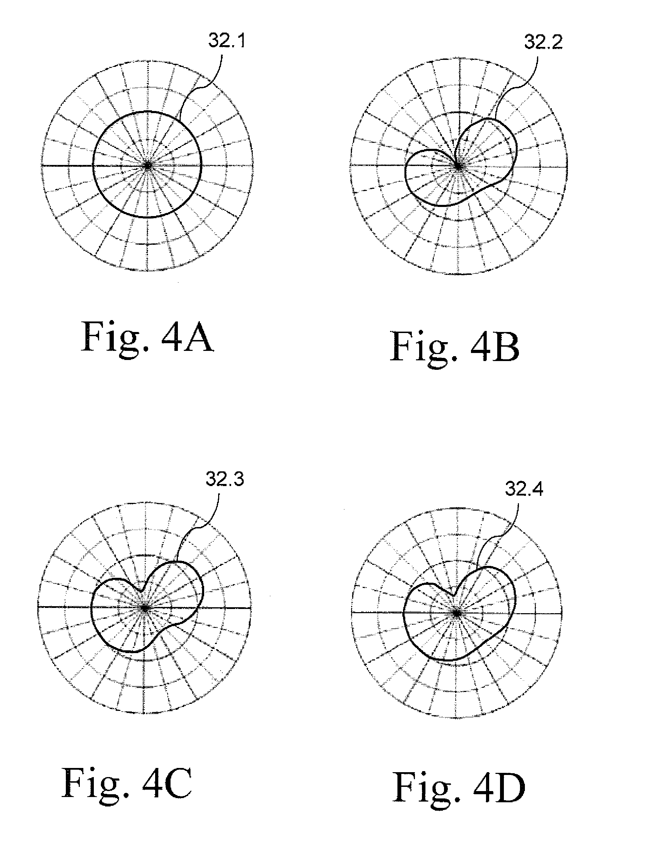

Das entsprechende Muster der normierten Daten, das sich für die Atmosphäre mit reiner Hauptkomponente (z.B. reiner Luft ohne zusätzliches Gas) ergibt, ist also konstant (Konstante 42 in Fig. 2) oder in einer polaren Darstellung kreisförmig. Das entsprechende Polardiagramme 34 mit einem solchen kreisförmigen Muster 32.1 ist in Fig. 4A dargestellt.

Die Figuren 4B - 4D zeigen Polardiagramme 34 mit weiteren Mustern 32.2 bis 32.4. Dabei entspricht das in dem Polardiagramm 34 der Fig. 4B dargestellte Muster 32.2 den normierten Daten bei einer den Sensor 12 umgebenden Atmosphäre aus Luft und einer geringen Beimengung von Ethanol (Alkohol). Das in dem Polardiagramm 34 der Fig. 4C dargestellte Muster 32.3 entspricht den normierten Daten bei einer den Sensor 12 umgebenden Atmosphäre aus Luft und einer geringen Beimengung von Feuerzeugbenzin (Leitbenzin) und das in dem Polardiagramm 34 der Fig. 4D dargestellte Muster 32.4 entspricht den normierten Daten bei einer den Sensor 12 umgebenden Atmosphäre aus Luft und einer geringen Beimengung von Zigarettenrauch. Jedes dieser Muster hat eine charakteristische Form, die Rückschlüsse auf das zu detektierende Gas (hier also Alkohol, Feuerzeugbenzin oder Zigarettenrauch) zulässt.

In Abhängigkeit von der Konzentration des zu detektierenden oxidierbaren und/oder reduzierbaren Gases ändert sich die (charakteristische) Form des Musters 32 nicht, wohl aber die Intensität der Messgröße (hier der normierten Daten). Diese Änderung der Intensität mit der Konzentration ist in erster Näherung in logarithmischer Darstellung linear, was durch die Gerade 44 in dem Diagramm 28 angedeutet ist.

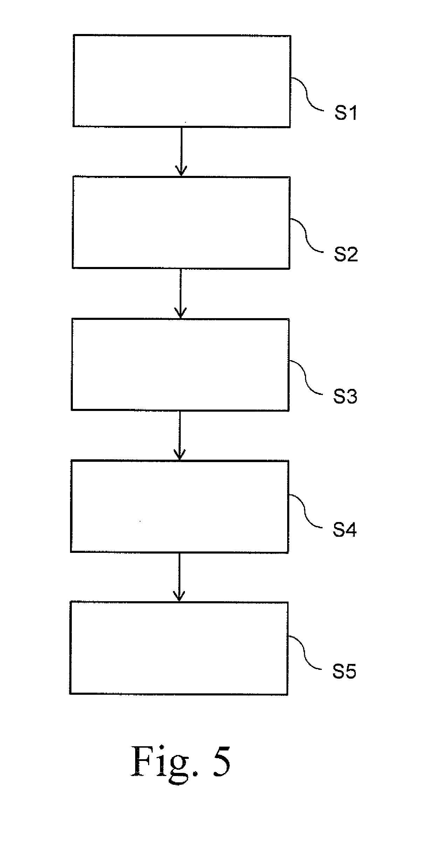

Fig. 5 zeigt schließlich einen Programmablaufplan eines Verfahrens zur Detektion mindestens eines oxidierbaren und/oder reduzierbaren Gases in einer eine Sensoroberfläche eines Halbleitergassensors umgebenden Atmosphäre, die neben diesem mindestens einen zu detektierenden Gas auch ein weiteres Gas oder Gasgemisch (z.B. Luft) als Hauptkomponente der Atmosphäre aufweist. Das Verfahren weist folgende Schritte auf:
Schritt 1 (S1): Datenaufnahme einer Sensor-Messgröße des Halbleitergassensors 12 bei mehreren mittels der Temperiereinrichtung eingestellten Sensortemperaturen in einem vorbestimmten Temperaturbereich, wobei der Halbleitergassensor der Atmosphäre mit dem mindestens einen oxidierbaren und/oder reduzierbaren Gas sowie dem die Hauptkomponente der Atmosphäre bildenden weiteren Gas oder Gasgemisch (z.B. Luft) ausgesetzt ist;
Schritt 2 (S2): Normieren der aufgenommenen Daten mittels einer bereitgestellten temperaturabhängigen Normierungsgröße,
Schritt 3 (S3): Vergleich der so normierten Daten mit mindestens einem Datensatz normierter Daten der Sensor-Messgröße bei entsprechenden Sensortemperaturen für eine Atmosphäre mit mindestens einen oxidierbaren und/oder reduzierbaren Referenzgas bekannter Konzentration sowie dem die Hauptkomponente der Atmosphäre bildenden weiteren Gas oder Gasgemisch (z.B. Luft) und
Schritt 4 (S4): Detektion des mindestens einen zu detektierenden Gases aufgrund der Vergleichsergebnisse als das Referenzgas oder zumindest eines der Referenzgase.

In einem optionalen Schritt 5 (S5) wird, nachdem das Gas erkannt worden ist (qualitative Messung), auch die tatsächliche Gaskonzentration (quantitative Messung) des Gases bestimmt oder zumindest abgeschätzt.

Die normierte (also relative) Messgröße R_{S}/R₀ ist im vorliegenden Fall eine mittels Ladungsträger-Transferverfahren ermittelte normierte Messgröße.

Referenzgase sind zum Beispiel: Aceton, Alkohol, Zigarettenrauch, etc. Die Datensätze mit den normierten Daten der Sensor-Messgröße bei entsprechenden Sensortemperaturen für eine Atmosphäre mit den unterschiedlichen oxidierbaren und/oder reduzierbaren Referenzgasen bekannter Konzentration werden dann einmalig mit einem baugleichen Sensor bestimmt und den einzelnen Gas-Sensorvorrichtungen zur Verfügung gestellt.

Generell ist es so, dass die Steigung der normierten Messwerte R_{S}/R₀ (normierten Daten) eine geringe Produktionsstreuung aufweist, wogegen die absoluten Messwerte Rs, Ro (absolut aufgenommene Daten) sehr stark streuen. Genauso wie bei Langzeitdrift, die Steigung (Empfindlichkeit) verändert sich kaum wogegen die absoluten Messwerte sehr wohl abdriften und zwar in nicht vorhergesehene Richtung.

Daher kann eine Mustererkennung mittels normierter Messwerte von einem Sensor 12 auf einen anderen Sensor 12 der gleichen Bauform übertragen werden. Die unterschiedlichen Chargen der Sensoren 12 spielen dabei kaum eine Rolle. Natürlich müssen die Sensoren 12 der gleichen Bauart entsprechen, zwischen verschiedenen Sensormodellen funktioniert die Normierung zumeist nicht.

D.h. wenn die Gasabdrücke mit einem Sensor 12 oder einem Sensorarray aufgenommen sind können diese einfach auf die anderen Sensoren 12 oder Sensorarrays mit anderen aus der gleichen Baureihe Sensoren 12 übertragen werden. Ein separater "Teaching-In" Prozess für jeden individuellen Sensor 12 ist dadurch nicht mehr notwendig.

Diese Eigenschaften können ebenso verwendet werden, um die Langzeitdrift der absoluten Werte für die Angaben der Gaskonzentration (quantitative Messung) automatisch entgegenzuwirken. Da das relative Verhalten der Sensoren 12 mit der Zeit auch recht stabil bleibt, gibt es immer noch die Problematik der Absolutdrift. Normalerweise wird dieser Absolutdrift durch einen Offset entgegengewirkt. Die Vorrichtung 10 muss aber dazu wissen wann genau dieses Offset gesetzt werden kann. In der Praxis wird die Null-Luft von einem Nutzer als "Luft ohne zusätzliches Gas" definiert und fordert das System manuell auf, den Offset zu setzen.

Da bei der Gas-Sensorvorrichtung 10 mittels Gasmustererkennung die reine Luft genau detektiert werden kann, ist es ebenso möglich im gleichen Vorgang den absoluten Wert zu ermittelt und den Offset zu definieren, falls die gespeicherten Werte zu sehr abweichen würden. Somit wäre dies die automatische Base-Level-Nachjustierung und die Vorrichtung 10 kann ohne Drift arbeiten.

Ergänzend sei darauf hingewiesen, dass bei dieser Anmeldung die Begriffe "aufweisend" und "umfassend" keine anderen Elemente oder Schritte ausschließt und die Verwendung des Artikels "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

### Bezugszeichen:

- 10: Gas-Sensorvorrichtung
- 12: Halbleitergassensor
- 14: Auswerteeinrichtung
- 16: Auswerteschaltung
- 18: Mikrocontroller
- 20: Strompfad
- 22: Referenzkondensator
- 24: Port
- 26: Port
- 28: Diagramm
- 30: Temperaturmodulationsschleife
- 32: Muster
- 34: Polardiagramm
- 36: Funktionsgraph, erster
- 38: Funktionsgraph, zweiter
- 40: Funktionsgraph, dritter
- 42: Konstante
- 44: Gerade
- S1: Schritt
- S2: Schritt
- S3: Schritt
- S4: Schritt
- S5: Schritt

## Patentansprüche

1. Verfahren zur Detektion mindestens eines oxidierbaren und/oder reduzierbaren Gases in einer eine Sensoroberfläche eines Halbleitergassensors (12), insbesondere eines Metalloxid-Halbleitergassensors, umgebenden Atmosphäre, die neben diesem mindestens einen zu detektierenden Gas auch ein weiteres Gas oder Gasgemisch als Hauptkomponente der Atmosphäre aufweist, **gekennzeichnet durch** die Schritte:
- Datenaufnahme einer Sensor-Messgröße des Halbleitergassensors (12) bei mehreren unterschiedlichen Sensortemperaturen in einem vorbestimmten Temperaturbereich, wobei der Halbleitergassensor (12) der Atmosphäre mit dem mindestens einen oxidierbaren und/oder reduzierbaren Gas sowie dem die Hauptkomponente der Atmosphäre bildenden weiteren Gas oder Gasgemisch ausgesetzt ist (S1),
- Normieren der aufgenommenen Daten mittels einer bereitgestellten temperaturabhängigen Normierungsgröße (S2),
- Vergleich der so normierten Daten mit mindestens einem Datensatz normierter Daten der Sensor-Messgröße bei entsprechenden Sensortemperaturen für eine Atmosphäre mit mindestens einem oxidierbaren und/oder reduzierbaren Referenzgas bekannter Konzentration sowie dem die Hauptkomponente der Atmosphäre bildenden weiteren Gas oder Gasgemisch (S3) und
- Detektion des mindestens einen zu detektierenden Gases aufgrund der Vergleichsergebnisse als das Referenzgas oder zumindest eines der Referenzgase (S4).

2. Verfahren nach Anspruch 1, **dadurch gekennzeichnet, dass** die bereitgestellte temperaturabhängige Normierungsgröße zuvor durch die folgenden Schritte ermittelt wird:
- Datenaufnahme einer Sensor-Messgröße bei unterschiedlichen Sensortemperaturen des Halbleitergassensors (12) oder eines baugleichen Halbleitergassensors in dem vorbestimmten Temperaturbereich, wobei der Halbleitergassensor der Atmosphäre mit dem reinen weiteren Gas oder Gasgemisch ausgesetzt ist, das die Hauptkomponente der Atmosphäre bildet, und
- Normieren der aufgenommenen Daten auf einen temperatur- und luftfeuchteunabhängigen konstanten Wert unter Ermittlung der dazu notwendigen temperaturabhängigen Normierungsgröße.

3. Verfahren nach einem der vorhergehenden Ansprüche, **dadurch gekennzeichnet, dass** die unterschiedlichen Sensortemperaturen insbesondere durch ein- oder mehrmaliges Durchlaufen mindestens einer Temperaturmodulationsschleife (30) eingestellt werden.

4. Verfahren nach einem der vorhergehenden Ansprüche, **dadurch gekennzeichnet, dass** bei der Detektion des mindestens einen zu detektierenden Gases aufgrund der Vergleichsergebnisse auch dessen Konzentration bestimmt oder zumindest abgeschätzt wird (S5).

5. Verfahren nach einem der vorhergehenden Ansprüche, **dadurch gekennzeichnet, dass** die Sensor-Messgröße eine in dem Halbleitergassensor (12) befindliche Ladungsmenge oder ein elektrischer Widerstand (Rs) des Halbleitergassensors ist.

6. Verfahren nach einem der vorhergehenden Ansprüche, **dadurch gekennzeichnet, dass** die Messgröße eine mittels Ladungsträger-Transferverfahren ermittelte Messgröße ist.

7. Verfahren nach einem der vorhergehenden Ansprüche, **dadurch gekennzeichnet, dass** eine Datenaufnahme bei Atmosphäre mit dem reinen weiteren Gas oder Gasgemisch an einem temperatur- und luftfeuchteunabhängigen konstanten Wert der normierten Daten erkannt wird und der Halbleitergassensor (12) durch Anpassung der Normierungsgröße mittels dieser Daten kalibriert wird.

8. Verfahren nach einem der Ansprüche 2 bis 7, **dadurch gekennzeichnet, dass** Ermittlung der temperaturabhängigen Normierungsgröße eine Ermittlung vor Erstinbetriebnahme einer den Halbleitergassensor (12) aufweisenden Gas-Sensorvorrichtung (10) ist.

9. Gas-Sensorvorrichtung (10) mit mindestens einem Halbleitergassensor (12), insbesondere Metalloxid-Halbleitergassensor, zur Detektion mindestens eines oxidierbaren und/oder reduzierbaren Gases in einer die Sensoroberfläche des Halbleitergassensors (12) umgebenden Atmosphäre, mit einer Temperiereinrichtung, insbesondere einer Heizung, zum gezielten Einstellen einer Sensortemperatur des Halbleitergassensors (12) innerhalb eines Temperaturbereichs und mit einer eine Auswerteschaltung (16), einen Prozessor und einen Speicher aufweisenden Auswerteeinrichtung (14) zur Auswertung einer Messgröße des Halbleitergassensors (12), wobei die Gas-Sensorvorrichtung (10) insbesondere zur Detektion gemäß eines Verfahrens nach mindestens einem der vorhergehenden Ansprüche eingerichtet ist.

10. Vorrichtung nach Anspruch 9, **dadurch gekennzeichnet, dass** die Temperiereinrichtung dazu eingerichtet ist, die Sensortemperatur des Halbleitergassensors (12) in einer Temperaturmodulationsschleife (30) zu durchfahren.

11. Vorrichtung nach Anspruch 9 oder 10, **dadurch gekennzeichnet, dass** die im Halbleitersensor (12) befindliche elektrische Ladungsmenge als Maß für die Konzentration der in der den mindestens einen Sensor (12) umgebenden Atmosphäre vorhandenen oxidierbaren oder reduzierbaren Gase genutzt und mittels der Auswerteeinrichtung (14) auswertbar ist.

12. Vorrichtung nach einem der Ansprüche 9 bis 11, **dadurch gekennzeichnet, dass** die Auswerteeinrichtung (14) einen Mikrocontroller (18), insbesondere einen für die Durchführung des Ladungsträger-Transferverfahren ausgelegten Mikrokontroller mit einer Schnittstelle für den direkten Anschluss des mindestens einen Halbleitergassensors (12), aufweist, wobei dieser Mikrocontroller (18) den Prozessor umfasst.
